(19) Europäisches Patentamt
European Patent Office
Office européen des brevets

(11) **EP 1 025 168 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**18.08.2010 Patentblatt 2010/33**

(45) Hinweis auf die Patenterteilung:
**18.12.2002 Patentblatt 2002/51**

(21) Anmeldenummer: **98951520.0**

(22) Anmeldetag: **14.10.1998**

(51) Int Cl.:
*C09C 1/00* (2006.01)   *C09D 7/12* (2006.01)
*C08K 3/00* (2006.01)   *C04B 33/14* (2006.01)
*C03C 4/02* (2006.01)   *C09D 11/00* (2006.01)

(86) Internationale Anmeldenummer:
**PCT/EP1998/006508**

(87) Internationale Veröffentlichungsnummer:
**WO 1999/020695 (29.04.1999 Gazette 1999/17)**

(54) **INTERFERENZPIGMENTE**

INTERFERENCE PIGMENTS

PIGMENTS INTERFERENTIELS

(84) Benannte Vertragsstaaten:
**DE ES FI FR GB IT**

(30) Priorität: **17.10.1997 DE 19746067**

(43) Veröffentlichungstag der Anmeldung:
**09.08.2000 Patentblatt 2000/32**

(73) Patentinhaber: **Merck Patent GmbH**
**64293 Darmstadt (DE)**

(72) Erfinder:
• **SCHMIDT, Christoph**
**D-65830 Kriftel (DE)**
• **PFAFF, Gerhard**
**D-64839 Münster (DE)**
• **SCHANK, Christina**
**D-64367 Mühetal (DE)**
• **SCHOEN, Sabine**
**D-64287 Darmstadt (DE)**

(56) Entgegenhaltungen:
| EP- - 0 753 545 | WO- -93//08237 |
|---|---|
| WO- -94//01498 | WO- -98//12266 |
| WO- -98//53011 | WO-A1-94/01498 |
| WO-A1-98/12266 | JP- - 7- 246 366 |
| US-A- 3 767 443 | US-A- 4 017 326 |
| US-A- 4 168 986 | US-A- 4 168 986 |

• **1996, CURT R.VINCENTZ VERLAG, HANNOVER Artikel R.GLAUSCH ET AL.: 'Perglanzpigmente', Seiten 1 - 53**
• **C.SCHMIDT ET AL.: 'Kontakte', 1992, DARMSTADT Seiten 15 - 24**

Bemerkungen:
Die Akte enthält technische Angaben, die nach dem Eingang der Anmeldung eingereicht wurden und die nicht in dieser Patentschrift enthalten sind.

EP 1 025 168 B2

**Beschreibung**

Die vorliegende Erfindung betrifft Interferenzpigmente auf der Basis von mehrfach beschichteten plättchenförmigen Substraten.

Glanz- oder Effektpigmente werden in vielen Bereichen der Technik eingesetzt, insbesondere im Bereich der Autolacke, der dekorativen Beschichtung, im Kunststoff, in Farben, Druckfarben sowie in kosmetischen Formulierungen.

Glanzpigmente, die einen winkelabhängigen Farbwechsel zwischen mehreren Interferenzfarben zeigen, sind aufgrund ihres Farbenspiels von besonderem Interesse für Autolacke sowie bei fälschungssicheren Wertschriften. Derartige Pigmente sind auf Basis von mehrfach beschichteten plättchenförmigen Substraten bekannt.

Interferenzpigmente bestehen in der Regel aus 200 bis 1000 nm dicken plättchenförmigen Substraten, die mit 50 bis 300 nm dicken, stark lichtbrechenden Metalloxiden bzw. Metalloxidgemischen überzogen sind. Die optischen Eigenschaften dieser Pigmente werden in entscheidendem Maße durch den Brechungsindex der Metalloxidschicht bestimmt. Neben der Möglichkeit über Chemical Vapor Deposition (CVD)- oder Physical Vapor Deposition (PVD)-Verfahren Metalloxidschichten herzustellen, die hohe Dichten und damit nahe am Optimum liegende Brechungsindizes aufweisen, erfolgt die Abscheidung von Metalloxiden auf feinteiligen, plättchenförmigen Substraten häufig durch Titration von wäßrigen, meist sauren Metallsalzlösungen gegen Natronlauge in Gegenwart eines Substrates, wie z.B. in den DE 14 67 468 und DE 20 09 566 beschrieben.

Nachteil des Aufdampfverfahrens sind die damit verbundenen hohen Kosten, So ist aus der U.S. 4,434,010 ein mehrschichtiges Interferenzpigment bekannt bestehend aus einer zentralen Schicht eines reflektierenden Metalls, wie z.B. Aluminium, und alternierenden Schichten zweier transparenter dielektrischer Materialien mit hohem und niedrigem Brechungsindex, wie z.B., Titandioxid und Siliziumdioxid. Dieses Mehrschichtpigment wird vorzugsweise für fälschungssichere Wertpapiere verwendet.

Aus der JP H7-759 ist ein mehrschichtiges Interferenzpigment mit metallischem Glanz bekannt, wobei ein Substrat mit alternierenden Schichten von Titandioxid und Siliziumdioxid beschichtet ist. Als Substrat werden Aluminium-, Gold- oder Silberflakes, oder Plättchen aus Glimmer bzw. Glas, die mit Metallen beschichtet sind, eingesetzt. Der für Interferenzpigmente charakteristische und erwünschte Tiefeneffekt kann allerdings nicht erzeugt werden. Dafür ist die Totalreflexion des Lichtes an der den Kern bildenden Metallschicht verantwortlich. Der Interferenzeffekt bleibt deshalb auf die sich auf der Metallschicht befindlichen Schichten begrenzt. Darüber hinaus werden aufgrund der fehlenden Transparenz des Substrates die vielfältigen Kombinationsmöglichkeiten mit weiteren Pigmenten in anwendungstechnischen Formulierungen stark eingeschränkt.

In den US 3,438,796 und US 5,135,812 werden zum Beispiel metallische Glanzpigmente beschrieben, die einen zentralen opaken Aluminiumfilm aufweisen, der beidseitig alternierend mit dielektrischen niedrigbrechenden Filmen, wie z.B. Siliziumdioxid oder Magnesiumfluorid, und teilweise transparenten Metallfilmen, wie z.B. Chrom oder Aluminium, beschichtet ist. Aufgrund des Herstellungsverfahrens ist der zentrale Metallfilm dieser Pigmente nur an der Plättchenober- und -unterseite beschichtet, während die Seitenflächen Bruchkanten darstellen und zum Medium hin offen liegen.

Aus der DE 44 05 494, DE 44 37 753, DE 195 16 181 und DE 195 15 988 sind Glanzpigmente bekannt, die durch Beschichtung von Metallplättchen, insbesondere Aluminiumflakes, über CVD-Verfahren oder naßchemisch mit niedrigbrechenden Metalloxidschichten, wie z.B. einer Siliziumdioxidschicht, und nicht selektiv absorbierenden hochbrechenden Metalloxidschichten bzw. Metallschichten hergestellt werden.

Glanzpigmente auf Basis von Metallsubstraten weisen häufig gute Anwendungseigenschaften, u.a. ein gutes Deckvermögen auf, jedoch resultiert bei der Applikation; z.B. im Lack, ein "harter" metallischer Glanz, der häufig nicht erwünscht ist.

Glanzpigmente auf Basis transparenter plättchenförmiger Substrate, die diesen "harten" metallischen Glanz nicht aufweisen, sind Gegenstand der WO 93/12182. Glimmerplättchen werden mit einer hochbrechenden Metalloxidschicht, wie z.B. $TiO_2$, und einer nicht selektiv absorbierenden Schicht belegt. Diese Pigmente zeigen in Abhängigkeit von der $TiO_2$-Schichtdicke in der Aufsicht eine bestimmte Interferenzfarbe, die mit schräger werdendem Blickwinkel zunehmend schwächer wird und schließlich nach grau bzw. schwarz abkippt. Die Interferenzfarbe ändert sich nicht, aber es ist eine Abnahme der Farbsättigung festzustellen.

In der JP 1992/93206 werden Glanzpigmente auf Basis von Glasflakes bzw. Glimmerpartikeln beansprucht, die mit einer opaken Metallschicht und alternierenden $SiO_2$- und $TiO_2$-Schichten belegt sind.

Aus der EP 0 753 545 sind Glanzpigmente auf Basis von mehrfach beschichteten, hochbrechenden, für sichtbares Licht zumindest teilweise durchlässigen, nichtmetallischen, plättchenförmigen Substraten bekannt, die mindestens ein Schichtpaket aus einer farblosen niedrigbrechenden und einer reflektierenden, selektiv oder nicht selektiv absorbierenden Beschichtung aufweisen. Nachteile dieser Erfindung sind der technisch sehr aufwendige und kostenintensive Herstellungsprozeß sowie die oft schwere Reproduzierbarkeit der Pigmente in der gewünschten Produktqualität.

Aufgabe der vorliegenden Erfindung ist es, ein im wesentlichen transparentes Interferenzpigment mit kräftigen Interferenzfarben und/oder einer starken Winkelabhängigkeit der Interferenzfarben zur Verfügung zu stellen, das sich durch seine vorteilhaften Anwendungseigenschaften auszeichnet und gleichzeitig auf einfache Art und Weise hergestellt werden kann.

Überraschenderweise wurde nun ein Interferenzpigment auf Basis von mehrfach beschichteten plättchenförmigen Substraten gefunden, das eine bestimmte Anordnung optisch funktioneller Schichten enthält, wodurch besondere optische Effekte erzielt werden.

Gegenstand der Erfindung sind somit Interferenzpigmente auf der Basis von mehrfach beschichteten plättchenförmigen Substraten, gemäß Anspruch 1.

Gegenstand der Erfindung ist weiterhin die Verwendung der erfindungsgemäßen Pigmente in Farben, Lacken, Druckfarben, Kunststoffen, keramischen Materialien, Gläsern und kosmetischen Formulierungen.

Geeignete Basissubstrate für die erfindungsgemäßen Mehrschichtpigmente sind einerseits opake und andererseits transparente plättchenförmige Substrate. Bevorzugte Substrate sind Schichtsilikate sowie mit Metalloxiden beschichtete plättchenförmige Materialien. Insbesondere geeignet sind natürliche und synthetische Glimmer, Talkum, Kaolin, plättchenförmige Eisenoxide, Bismutoxidchlorid, Glas-, $SiO_2$-, $Al_2O_3$-, $TiO_2$oder synthetische Keramikflakes, synthetische trägerfreie Plättchen, LCPs oder andere vergleichbare Materialien.

Die Größe der Basissubstrate ist an sich nicht kritisch und kann auf den jeweiligen Anwendungszweck abgestimmt werden. In der Regel haben die plättchenförmigen Substrate eine Dicke zwischen 0,1 und 5 $\mu$m, insbesondere zwischen 0,2 und 4,5 $\mu$m. Die Ausdehnung in den beiden anderen Bereichen beträgt üblicherweise zwischen 1 und 250 $\mu$m, vorzugsweise zwischen 2 und 200 $\mu$m and insbesondere zwischen 5 und 50 $\mu$m.

Die Dicke der einzelnen Schichten mit hohem und niedrigem Brechungsindex auf dem Basissubstrat ist wesentlich für die optischen Eigenschaften des Pigments. Für ein Pigment mit intensiven Interferenzfarben, muß die Dicke der einzelnen Schichten genau aufeinander eingestellt werden.

Wenn n der Brechungsindex einer dünnen Schicht und d ihre Dicke ist, wird die Interferenzfarbe dieser Schicht von dem Produkt n·d (n·d = optische Dicke) bestimmt. Die bei Normallichteinfall im reflektierten Licht entstehenden Farben eines solchen Films ergeben sich aus einer Verstärkung des Lichtes der Wellenlänge

$$\lambda = \frac{4}{2N-1} \cdot n \cdot d$$

und durch Schwächung des Lichtes der Wellenlänge,

$$\lambda = \frac{2}{N} \cdot n \cdot d$$

wobei N eine positive ganze Zahl ist.

Die bei zunehmender Filmdicke resultierende Variation der Farbe ergibt sich aus der Verstärkung bzw. Schwächung bestimmter Wellenlängen des Lichtes durch Interferenz. Wenn mehrere Schichten in einem Mehrschichtpigment die gleiche optische Dicke besitzen, wird die Farbe des reflektierten Lichts mit zunehmender Zahl der Schichten intensiver. Darüber hinaus kann durch geeignete Wahl der Schichtdicken eine besonders starke Variation der Farbe in Abhängigkeit vom Betrachtungswinkel erreicht werden. Es bildet sich ein ausgeprägter sogenannter Farbflop aus. Die Dicke der einzelnen Metalloxidschichten unabhängig von ihrem Brechungsindex beträgt in Abhängigkeit vom Anwendungsgebiet in der Regel 10 bis 1000 nm, vorzugsweise 15 bis 800 nm und insbesondere 20-600 nm.

Die erfindungsgemäßen Glanzpigmente weisen eine hochbrechende Beschichtung (A) in Kombination mit einer farblosen niedrigbrechenden Beschichtung (B) und einer darauf befindlichen nicht absorbierenden hochbrechenden Beschichtung auf. Die Pigmente können mehrere, gleiche oder verschiedene Kombinationen an Schichtpaketen enthalten, bevorzugt ist aber die Belegung des Substrats mit nur einem Schichtpaket (A) + (B) + (C). Zur Intensivierung des Farbflops kann das erfindungsgemäße Pigment bis zu 4 Schichtpakete enthalten, wobei die Dicke aller Schichten auf dem Substrat 3 $\mu$m allerdings nicht überschreiten sollte.

Die hochbrechende Schicht (A) hat einen Brechungsindex n ≥ 2,0, vorzugsweise n ≥ 2,1, welche ganz oder im wesentlichen aus Eisenoxid besteht.

Die Dicke der Schicht (A) beträgt 20 - 350 nm.

Als farblose niedrigbrechende für die Beschichtung (B) geeignete Materialien sind vorzugsweise Metalloxide bzw. die entsprechenden Oxidhydrate, wie z.B. $SiO_2$, $Al_2O_3$, $AIO(OH)$, $B_2O_3$ oder ein Gemisch der genannten Metalloxide, geeignet. Die Dicke der Schicht (B) beträgt 30 - 600 nm.

Für die nicht absorbierende hochbrechende Beschichtung (C) eignen sich insbesondere farblose Metalloxide wie $TiO_2$, $ZrO_2$, $SnO_2$, $ZnO$ und $BiOCl$ sowie deren Gemische. Die Dicke der Schicht (C) beträgt 20 - 350 nm.

Neben dem Standardschichtpaket (A) + (B) + (C), das das erfindungsgemäße Pigment bis zu viermal aufweisen kann, gibt es weitere bevorzugte Ausführungsformen. So kann das erfindungsgemäße Pigment zwischen dem Substrat (S) und der Schicht (A), zwischen der Schicht (A) und (B), der Schicht (B) und (C) und/oder der Schicht (C) und der Deckschicht (D) eine weitere absorbierende oder nicht absorbierende Schicht [(S1), (A1), (B1), (C1)] aufweisen. Die Dicken der Zwischenschichten betragen 1 - 50 nm, vorzugsweise 1 - 40 nm und insbesondere 1 - 30 nm.

Eine besonders bevorzugte Ausführungsform ist die Beschichtung des Substrats mit folgendem Schichtpaket:

  (S1) optional, $SnO_2$
  (A) $Fe_2O_3$
  (B) $SiO_2$
  (B1) optional, $SnO_2$

(C) TiO$_2$

(D) anwendungsbezogene Nachbeschichtung

Durch die Beschichtung der Substrate mit hochbrechenden Schichten (A) und (C) und einer niedrigbrechenden Schicht (B) und gegebenenfalls weiteren farbigen oder farblosen Beschichtungen entstehen Pigmente, deren Farbe, Glanz, Deckvermögen und Winkelabhängigkeit des Farbeindruckes in weiten Grenzen variiert werden können.

Die erfindungsgemäßen Pigmente lassen sich leicht herstellen durch die Erzeugung mehrerer hoch und niedrig brechender Interferenzschichten mit genau definierter Dicke und glatter Oberfläche auf den feinteiligen, plättchenförmigen Substraten.

Die Metalloxidschichten werden vorzugsweise naßchemisch aufgebracht, wobei die zur Herstellung von Perlglanzpigmenten entwickelten naßchemischen Beschichtungsverfahren angewendet werden können; derartige Verfahren sind z.B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren Patentdokumenten und sonstigen Publikationen.

Bei der Naßbeschichtung werden die Substratpartikel in Wasser suspendiert und mit einem oder mehreren hydrolysierbaren Metallsalzen bei einem für die Hydrolyse geeigneten pH-Wert versetzt, der so gewählt wird, daß die Metalloxide bzw. Metalloxidhydrate direkt auf den Plättchen ausgefällt werden, ohne daß es zu Nebenfällungen kommt. Der pH-Wert wird üblicherweise durch gleichzeitiges Zudosieren einer Base und/oder Säure konstant gehalten. Anschließend werden die Pigmente abgetrennt, gewaschen und getrocknet und gegebenenfalls geglüht, wobei die Glühtemperatur im Hinblick auf die jeweils vorliegende Beschichtung optimiert werden kann. In der Regel liegen die Glühtemperaturen zwischen 250 und 1000 °C, vorzugsweise zwischen 350 und 900 °C. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Perlglanzpigmenten vorgeschlagenen Verfahren entsprechend angewendet werden können.

Als Metalloxid mit einem hohen Brechungsindex wird bevorzugt Eisenoxid und als Metalloxid mit niedrigem Brechungsindex Siliziumdioxid verwendet.

Für das Aufbringen der Titandioxidschichten wird das im US 3,553,001 beschriebene Verfahren bevorzugt.

Zu einer auf etwa 50-100 °C erhitzten Suspension des zu beschichtenden Materials wird langsam eine wäßrige Titansalzlösung zugegeben, und es wird durch gleichzeitiges Zudosieren einer Base, wie z.B. wäßrige Ammoniaklösung oder wäßrige Alkalilauge, ein weitgehend konstanter pH-Wert von etwa 0,5-5 eingehalten. Sobald die gewünschte Schichtdicke der TiO$_2$-Fällung erreicht ist, wird die Zugabe der Titansalzlösung und der Base gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO$_2$ erforderlich ist und wie pro Zeiteinheit von der verfügbaren Oberfläche der zu beschichtenden Teilchen aufgenommen werden kann. Es entstehen deshalb keine hydratisierten Titandioxidteilchen, die nicht auf der zu beschichtenden Oberfläche niedergeschlagen sind.

Das Aufbringen der Siliziumdioxidschichten kann z. B. wie folgt vorgenommen werden. Zu einer auf etwa 50-100 °C erhitzten Suspension des zu beschichtenden Substrats wird eine Kali- oder Natronwasserglaslösung, zudosiert. Durch gleichzeitige Zugabe einer verdünnten Mineralsäure, wie z. B. HCl, HNO$_3$ oder H$_2$SO$_4$, wird der pH-Wert bei ca. 6 - 9 konstant gehalten. Sobald die gewünschte Schichtdicke an SiO$_2$ erreicht ist, wird die Zugabe der Wasserglaslösung gestoppt. Anschließend wird ca. 0,5 h nachgerührt.

Zur Erhöhung der Licht- und Wetterstabilität empfiehlt es sich häufig, in Abhängigkeit vom Einsatzgebiet das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische Stabilität weiter erhöht oder die Handhabung des Pigments, insbesondere die Einarbeitung in unterschiedliche Medien, erleichtert.

Die erfindungsgemäßen Pigmente sind mit einer Vielzahl von Farbsystemen kompatibel vorzugsweise aus dem Bereich der Lacke, Farben und Druckfarben, insbesondere Sicherheitsdruckfarben. Aufgrund der nicht kopierbaren optischen Effekte können die erfindungsgemäßen Pigmente insbesondere bei der Herstellung von fälschungssicheren Wertschriften, wie z. B. Geldscheine, Schecks, Scheckkarten, Kreditkarten, Ausweisen, etc., verwendet werden. Ferner sind die Pigmente auch für die Lasermarkierung von Papier und Kunststoffen sowie für Anwendungen im Agrarbereich, z.B. für Gewächshausfolien, geeignet.

Gegenstand der Erfindung ist somit auch die Verwendung der Pigmente in Formulierungen wie Farben, Druckfarben, Lacken, Kunststoffen, keramischen Materialien, Gläsern und zur Kosmetikpräparation.

Es versteht sich von selbst, daß für die verschiedenen Anwendungszwecke die Mehrschichtpigmente auch vorteilhaft in Abmischung mit anderen Pigmenten, wie z. B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und $SiO_2$-Plättchen, etc., verwendet werden können. Die Mehrschichtpigmente können in jedem Verhältnis mit handelsüblichen Pigmenten und Füllern gemischt werden.

Die nachfolgenden Beispiele sollen die Erfindung näher erläutern ohne sie jedoch zu beschränken.

Beispiele

Beispiel 1

100 g Glimmer (PSD 10-60 $\mu$m) werden in 2 l vollentsalztem Wasser auf 80 °C erhitzt. Bei dieser Temperatur werden unter kräftigem Rühren 430 g Eisen-III-chlorid-Lösung (14,25 % Fe) zudosiert. Dabei wird mit Natronlauge (32 % NaOH) der pH-Wert konstant bei 4,0 gehalten. Anschließend wird der pH-Wert mit Salzsäure (15 % HCl) auf 1,8 abgesenkt und bei diesem pH-Wert 30 ml $TiCl_4$-Lösung (400 g $TiCl_4$/l) zugegeben. Der pH-Wert wird dabei mit Natronlauge (32 % NaOH) konstant gehalten. Anschließend wird der pH-Wert mit Natronlauge (32 % NaOH) auf 7,5 angehoben und bei diesem pH eine Lösung von 252 g Natronwasserglas (27 % $SiO_2$) in 252 g vollentsalztem Wasser zugegeben. Der pH Wert wird dabei mit Salzsäure (15 % HCl) konstant gehalten.

Danach wird der pH-Wert mit Salzsäure (15 % HCl) auf 2,0 abgesenkt und bei diesem pH-Wert eine Lösung von 3 g $SnCl_4$ x 5 $H_2O$ und 10 ml Salzsäure (37 % HCl) in 90 ml vollentsalztem Wasser zudosiert. Der pH-Wert wird dabei mit Natronlauge (32 % NaOH) konstant gehalten. Anschließend wird der pH-Wert mit Salzsäure (15 % HCl) auf 1,8 abgesenkt und bei diesem pH-Wert 655 ml $TiCl_4$-Lösung (400 g/l) zudosiert. Dabei wird der pH-Wert mit Natronlauge (32 % NaOH) konstant gehalten. Nach Zugabe der $TiCl_4$-Lösung wird 15 Min. nachgerührt und das Produkt abfiltriert, mit vollentsalztem Wasser gewaschen, bei ca. 110 °C getrocknet und 45 Min. bei 850 °C geglüht. Das erhaltene Interferenzpigment zeichnet sich durch eine intensive rotviolette Interferenzfarbe aus.

Beispiel 2

100 g Glimmer (PSD 10-60 $\mu$m) werden in 2 l vollentsalztem Wasser auf 80 °C erhitzt. Bei dieser Temperatur werden unter kräftigem Rühren 430 g Eisen-III-chlorid-Lösung (14,25 % Fe) zudosiert. Dabei wird mit Natronlauge (32 % NaOH) der pH-Wert konstant bei 4,0 gehalten. Anschließend wird der pH-Wert mit Natronlauge (32 % NaOH) auf 7,5 angehoben und bei diesem pH eine Lösung von 252 g Natronwasserglas (27 % $SiO_2$) in 252 g vollentsalztem Wasser zugegeben. Der pH-Wert wird dabei mit Salzsäure (15 % HCl) konstant gehalten.

Danach wird der pH-Wert mit Salzsäure (15 % HCl) auf 2,0 abgesenkt und eine Lösung von 3 g $SnCl_4$ x 5 $H_2O$ und 10 ml Salzsäure (37 % HCl) in 90 ml vollentsalztem Wasser zudosiert. Der pH-Wert wird dabei mit Natronlauge (32 % NaOH) konstant gehalten. Anschließend wird der pH-Wert mit Salzsäure (15 % HCl) auf 1,8 abgesenkt und 476 ml $TiCl_4$-Lösung (400 g/l) zudosiert. Dabei wird der pH-Wert mit Natronlauge (32 % NaOH) konstant gehalten. Nach Zugabe der $TiCl_4$-Lösung wird 15 Min. nachgerührt und das Produkt abfiltriert, mit vollentsalztem Wasser gewaschen, bei 110 °C getrocknet und 30 Min. bei 850 °C geglüht. Das erhaltene Interferenzpigment zeichnet sich durch eine intensive rote Interferenzfarbe aus.

Beispiel 3

100 g Muskovit-Glimmer (Teilchengröße 10-60 $\mu$m) werden in 2 l vollentsalztem Wasser auf 80 °C erwärmt. Unter kräftigem Rühren wird dann eine Lösung von 3 g $SnCl_4$ x 5 $H_2O$ und 10 ml Salzsäure (37 % HCl) in 90 ml vollentsalztem Wasser bei pH 2,0 zugegeben. Der pH-Wert wird dabei mit Natronlauge (32 % NaOH) konstant gehalten. Anschließend werden bei pH 1,8 155 ml $TiCl_4$-Lösung (400 g/$TiCl_4$/l) zugegeben. Der pH-Wert wird dabei mit Natronlauge (32 % NaOH) konstant gehalten. Daraufhin wird der pH-Wert mit Natronlauge auf 2,6 erhöht und bei diesem pH-Wert 100 ml einer Lösung von 25 ml $TiCl_4$-Lösung (400 g $TiCl_4$/l), 48 g $FeCl_3$-Lösung (14,25 % Fe) und 4,8 g $AlCl_3$ x 6 $H_2O$ in vollentsalztem Wasser zugegeben. Dabei wird der pH-Wert mit Natronlauge (32 % NaOH) konstant gehalten.

Anschließend wird der pH-Wert mit Natronlauge (32 % NaOH) auf 7,5 erhöht und bei diesem pH-Wert eine Lösung von 271 g Natronwasserglas (27 % $SiO_2$) in 271 g vollentsalztem Wasser zudosiert. Der pH-Wert wird mit Salzsäure (10 % HCl) konstant gehalten. Anschließend wird der pH-Wert mit Salzsäure (10 % HCl) auf 2,0 abgesenkt und eine Lösung von 3 g $SnCl_4$ x 5 $H_2O$ und 10 ml Salzsäure (37 % NaOH) in 90 ml VE-Wasser zudosiert. Der pH-Wert wird dabei mit Natronlauge (32 % NaOH) konstant gehalten. Anschließend werden bei pH = 1,8 45 ml $TiCl_4$-Lösung (400 g $TiCl_4$/l) zugegeben, wobei der pH-Wert wiederum mit Natronlauge (32 % NaOH) konstant gehalten wird. Danach wird der pH-Wert mit Natronlauge (32 % NaOH) auf 2,6 erhöht und bei diesem pH-Wert 230 ml einer Lösung von 129 ml $TiCl_4$-Lösung (400 g $TiCl_4$/l), 206 g $FeCl_3$-Lösung (14,25 % Fe) und 10,2 g $AlCl_3$ x 6 $H_2O$ in 157 ml VE-Wasser zudosiert. Der pH-Wert wird dabei mit Natronlauge (32 % NaOH) konstant gehalten. Zuletzt wird das Pigment abgesaugt, mit vollentsalztem Wasser gewaschen, bei 110 °C getrocknet und 30 Min. bei 850 °C geglüht. Man erhält ein intensiv rotviolett gefärbtes Interferenzpigment, das beim Abkippen über orange in ein kräftiges gelbgrün übergeht.

Beispiel 4

100 g Muskovit-Glimmer (Teilchengröße 10 - 60 µm) werden in 2 l vollentsalztem Wasser auf 80 °C erwärmt. Unter kräftigem Rühren wird dann eine Lösung von 3 g $SnCl_4$ x 5 $H_2O$ und 10 ml Salzsäure (37 % HCl) in 90 ml vollentsalztem Wasser bei pH 2,0 mit einer Dosierrate von 4 ml/min, zugegeben. der pH-Wert wird dabei mit Natronlauge (32 % NaOH) konstant gehalten. Anschließend werden bei pH 1,8 155 ml $TiCl_4$-Lösung (400 g $TiCl_4$/l) mit einer Dosierrate von 2 ml/min, zugegeben. Der pH-Wert wird dabei mit Natronlauge (32 % NaOH) konstant gehalten. Daraufhin wird der pH-Wert mit Natronlauge auf 2,6 erhöht und bei diesem pH 100 ml einer Lösung von 25 ml $TiCl_4$-Lösung (400 g $TiCl_4$/l), 48 g $FeCl_3$-Lösung (14,25 % Fe) und 4,8 g $AlCl_3$ x 6 $H_2O$ in vollentsalztem Wasser zugegeben. Dabei wird der pH mit Natronlauge (32 % NaOH) konstant gehalten.

Anschließend wird der pH mit Natronlauge (32 % NaOH) auf 7,5 erhöht und bei diesem pH-Wert eine Lösung von 297 g Natronwasserglas (27 % $SiO_2$) in 297 g vollentsalztem Wasser mit einer Geschwindigkeit von 2 ml/min, zudosiert. Der pH-Wert wird mit Salzsäure (10 % HCl) konstant gehalten. Anschließend wird der pH-Wert mit Salzsäure (10 % HCl) auf 2,0 abgesenkt und eine Lösung von 3 g $SnCl_4$ x 5 $H_2O$ und 10 ml Salzsäure (37 % HCl) in 90 ml vollentsalztem Wasser mit einer Geschwindigkeit von 4 ml/min. zudosiert. Der pH-Wert wird dabei mit Natronlauge (32 % NaOH) konstant gehalten. Anschließend werden bei pH 1,8 250,5 ml $TiCl_4$-Lösung (400 g $TiCl_4$/l) mit einer Geschwindigkeit von 2 ml/min. zugegeben, wobei der pH-Wert wiederum mit Natronlauge (32 % NaOH) konstant gehalten wird.

Anschließend wird das Pigment abgesaugt, mit VE-Wasser gewaschen, bei 110 °C getrocknet. Das erhaltene Pigment besitzt nach dieser Stufe rotviolette Farbe, die beim Abkippen in Gelbgrün übergeht.

Abschließend wird bei 850 °C 30 Minuten geglüht. Man erhält ein gelbrot glänzendes Interferenzpigment, dessen Farbe beim Abkippen in gelbgrün übergeht.

Beispiel 5

Die Fällung der Metalloxidschichten wird analog Beispiel 5 durchgeführt. Zusätzlich werden bei pH-Wert 2,6 130 ml einer Mischung von 129 ml $TiCl_4$-Lösung (400 g $TiCl_4$/l), 147 ml $FeCl_3$-Lösung (14,08 % Fe), 10,2 g $AlCl_3$ x 6 $H_2O$ und 157 ml vollentsalztem Wasser mit einer Dosierrate von 1 ml/min, zugesetzt. Der pH-Wert wird dabei mit Natronlauge (32 % NaOH) konstant gehalten.

Die Aufarbeitung erfolgt wie bei den Beispielen 1 - 5.

Das getrocknete Pigment zeigt intensive rotviolette Farbe mit hohem Glanz, die beim Abkippen in orange übergeht. Nach Glühung glänzt das Pigment gelbrot, wobei die Farbe beim Abkippen in ein kräftig glänzendes Gelb übergeht.

**Patentansprüche**

1. Interferenzpigmente auf der Basis von mehrfach beschichteten plättchenförmigen Substraten, die mit mindestens einer Schichtenfolge mit optisch funktionellen Schichten aus

    (A) einer Beschichtung mit einem Brechungsindex n ≥ 2,0, welche ganz oder im wesentlichen aus Eisenoxid besteht, und einer Dicke von 20-350 nm,
    (B) einer farblosen Beschichtung mit einem Brechungsindex n ≤ 1,8, und einer Dicke von 30-600 nm und
    (C) einer nicht absorbierenden hochbrechenden Beschichtung mit einer Dicke von 20-350 nm,
    sowie gegebenenfalls
    (D) eine äußere Schutzschicht

    durch Nassbeschichtung oder Gasphasenbeschichtung im Wirbelbett umhüllt sind.

2. Interferenzpigmente nach Anspruch 1, **dadurch gekennzeichnet, dass** sich zwischen dem Substrat (S) und der Schicht (A), der Schicht (A) und (B), der Schicht (B) und (C) und/oder der Schicht (C) und (D) eine weitere farbige oder farblose Metalloxidschicht (S1), (A1), (B1) und/oder (C1) befindet.

3. Interferenzpigmente nach Anspruch 1 oder 2, **dadurch gekennzeichnet, dass** es sich bei den plättchenförmigen Substraten um natürlichen oder synthetischen Glimmer, Glas-, $Al_2O_3$-, $SiO_2$- oder $TiO_2$-Flakes sowie mit Metalloxiden beschichtete, plättchenförmige Materialien handelt.

4. Interferenzpigmente nach einem der Ansprüche 1 bis 3, **dadurch gekennzeichnet, dass** die Schichten (A), (B) und (C) im wesentlichen aus Metalloxiden besteht.

5. Interferenzpigmente nach einem der Ansprüche 1 bis 4, **dadurch gekennzeichnet, dass** die Schicht (B) im wesentlichen aus Siliziumdioxid, Aluminiumoxid, Magnesiumfluorid oder deren Gemischen besteht.

6. Interferenzpigmente nach einer der Ansprüche 1 bis 5, **dadurch gekennzeichent, dass** die Schicht (C) im wesentlichen aus Titandioxid, Bismutoxidchlorid, Zirkonoxid, Zinnoxid, Zinkoxid oder deren Gemischen besteht.

7. Interferenzpigmente nach einem der Ansprüche 1 bis 6, **dadurch gekennzeichnet, dass** sie bis zu viermal die Schichtenfolge (A) - (C) aufweisen.

8.  Interferenzpigmente nach Anspruch 7, **dadurch gekennzeichnet, dass** sie nur eine Schichtenfolge (A)-(C) enthalten.

9.  Verfahren zur Herstellung der Intereferenzpigmente nach einem der Ansprüche 1 bis 7, **dadurch gekennzeichnet, dass** die Metalloxide nasschemisch durch hydrolytische Zersetzung von Metallsalzen in wässrigen Medium auf das plättchenförmige Substrat aufgebracht werden.

10. Verwendung der Intereferenzpigmente nach Anspruch 1 in Farben, Lacken, Druckfarben, Kunststoffen, keramischen Materialien, Gläsern und in kosmetischen Formulierungen.

**Claims**

1.  Interference pigments based on multicoated flakeform substrates which are sheathed with at least one layer sequence having optically functional layers comprising

    (A) a coating having a refractive index n ≥ 2.0 which consists entirely or essentially of iron oxide, and a thickness of 20-350 nm,
    (B) a colourless coating having a refractive index n ≤ 1.8 and a thickness of 30-600 nm and
    (C) a non-absorbent, high-refractive-index coating having a thickness of 20-350 nm,
    and optionally
    (D) an outer protective layer,

    by wet coating or gas-phase coating in a fluidised bed.

2.  Interference pigments according to Claim 1, **characterised in that** a further coloured or colourless metal oxide layer (S1), (A1), (B1) and/or (C1) is located between the substrate (S) and layer (A), layers (A) and (B), layers (B) and (C) and/or layers (C) and (D).

3.  Interference pigments according to Claim 1 or 2, **characterised in that** the flake-form substrates are natural or synthetic mica, glass flakes, Al$_2$O$_3$ flakes, SiO$_2$ flakes or TiO$_2$ flakes or metal oxide-coated, flake-form materials.

4.  Interference pigments according to one of Claims 1 to 3, **characterised in that** layers (A), (B) and (C) essentially consist of metal oxides.

5.  Interference pigments according to one of Claims 1 to 4, **characterised in that** layer (B) essentially consists of silicon dioxide, aluminium oxide, magnesium fluoride or mixtures thereof.

6.  Interference pigments according to one of Claims 1 to 5, **characterised in that** layer (C) essentially consists of titanium dioxide, bismuth oxide chloride, zirconium oxide, tin oxide, zinc oxide or mixtures thereof.

7.  Interference pigments according to one of Claims 1 to 6, **characterised in that** they have the layer sequence (A) - (C) up to four times.

8.  Interference pigments according to Claim 7, **characterised in that** they comprise only one layer sequence (A) - (C).

9.  Process for the preparation of the interference pigments according to one of Claims 1 to 7, **characterised in that** the metal oxides are applied to the flakeform substrate by wet-chemical methods by hydrolytic decomposition of metal salts in aqueous medium.

10. Use of the interference pigments according to Claim 1 in paints, coatings, printing inks, plastics, ceramic materials, glasses and in cosmetic formulations.

**Revendications**

1.  Pigments d'interférence à base de substrats multicouches en forme de paillettes gainés par au moins une séquence de couches ayant des couches optiquement fonctionnelles comprenant

    (A) un revêtement ayant un indice de réfraction n ≥ 2,0 qui est constitué totalement ou essentiellement d'oxyde de fer, et ayant une épaisseur de 20-350 nm,
    (B) un revêtement incolore ayant un indice de réfraction n ≤ 1,8 et une épaisseur de 30-600 nm, et
    (C) un revêtement non-absorbant, à haut indice de réfraction, ayant une épaisseur de 20-350 nm,
    et éventuellement
    (D) une couche protectrice externe,

    par enduction par voie humide ou en phase gazeuse dans un lit fluidisé.

2.  Pigments d'interférence selon la revendication 1, **caractérisés en ce qu'**une couche supplémentaire d'oxyde métallique colorée ou incolore (S1), (A1), (B1) et/ou (C1) est disposée entre le substrat (S) et la couche (A), les couches (A) et (B), les couches (B) et (C) et/ou les couches (C) et (D).

3. Pigments d'interférence selon la revendication 1 ou 2, **caractérisés en ce que** les substrats en forme de paillettes sont du mica naturel ou synthétique, des paillettes de verre, des paillettes d'$Al_2O_3$, des paillettes de $SiO_2$ ou des paillettes de $TiO_2$ ou des matériaux en forme de paillettes revêtus d'oxyde métallique.

4. Pigments d'interférence selon l'une des revendications 1 à 3, **caractérisés en ce que** les couches (A), (B) et (C) sont constituées essentiellement d'oxydes métalliques.

5. Pigments d'interférence selon l'une des revendications 1 à 4, **caractérisés en ce que** la couche (B) est constituée essentiellement de dioxyde de silicium, d'oxyde d'aluminium, de fluorure de magnésium ou de mélanges de ceux-ci.

6. Pigments d'interférence selon l'une des revendications 1 à 5, **caractérisés en ce que** la couche (C) est constituée essentiellement de dioxyde de titane, de chlorure d'oxyde de bismuth, d'oxyde de zirconium, d'oxyde d'étain, d'oxyde de zinc ou de mélanges de ceux-ci.

7. Pigments d'interférence selon l'une des revendications 1 à 6, **caractérisés en ce qu'**ils comportent la séquence de couches (A) - (C) jusqu'à quatre fois.

8. Pigments d'interférence selon la revendication 7, **caractérisés en ce qu'**ils comportent la séquence de couches (A) - (C) une fois seulement.

9. Procédé de préparation des pigments d'interférence selon l'une des revendications 1 à 7, **caractérisé en ce que** les oxydes métalliques sont appliqués au substrat en forme de paillettes par des méthodes chimiques par voie humide, via une décomposition hydrolytique de sels métalliques en milieu aqueux.

10. Utilisation des pigments d'interférence selon la revendication 1 dans des peintures, des revêtements, des encres d'impression, des matières plastiques, des matériaux céramiques, des verres et dans des formulations cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1467468
- DE 2009566
- US 4434010 A
- JP H7759 A
- US 3438796 A
- US 5135812 A
- DE 4405494
- DE 4437753
- DE 19516181
- DE 19515988
- WO 9312182 A
- JP 4093206 A
- EP 0753545 A
- DE 1959988
- DE 2214545
- DE 2215191
- DE 2244298

- DE 2313331
- DE 2522572
- DE 3137808
- DE 3137809
- DE 3151343
- DE 3151354
- DE 3151355
- DE 3211602
- DE 3235017
- EP 0045851 A
- EP 0106235 A
- US 3553001 A
- DE 2215191 C
- DE 3151354 A
- DE 3235017 A
- DE 3334598 A